# EUROPEAN PATENT APPLICATION

(11) **EP 4 216 140 A1**
(43) Date of publication of application: **26.07.2023**
(21) Application number: 20954116.8
(22) Date of filing: 17.09.2020
(51) Int. Cl.: G06Q 30/06, A24F 40/65

(54) **SYSTEM, TERMINAL DEVICE, CONTROL METHOD, AND PROGRAM**

(71) Applicant: Japan Tobacco Inc., Tokyo 105-6927 (JP)
(72) Inventor: YAMADA, Manabu, Tokyo 130-8603 (JP)
(74) Representative: Hoffmann Eitle
(86) International application number: PCT/JP2020/035241
(87) International publication number: WO 2022/059130

(57) **Abstract**

[Problem] To provide a mechanism that can avoid an inexpedience that may occur when a plurality of accessories are replaced in an inhalation device.

[Solution] Provided is a system comprising a plurality of accessories which are detachable from an inhalation device that uses a base substance to generate aerosol to be inhaled by a user and include information read and written via wireless communication, and a terminal device that stores information to be synchronized that should be stored in common in the plurality of accessories, and writes the stored information to be synchronized to each of the plurality of accessories via the wireless communication.

## Description

### Technical Field

The present invention relates to systems, terminal devices, control methods, and programs.

### Background Art

Inhaler devices including electronic cigarettes and nebulizers that generate material to be inhaled by users are becoming widely popular. For example, an inhaler device uses a substrate, which includes an aerosol source for generating an aerosol and a flavor source for adding a flavor component to the generated aerosol, to generate a flavor-component-added aerosol. A user can taste the flavor by inhaling the flavor-component-added aerosol generated by the inhaler device.

In recent years, there have been studies for providing various types of inhaler-device-related services by wirelessly exchanging inhaler-device-related information. For example, Patent Literature 1 indicated below discloses technology in which a terminal at a store that sells substrates wirelessly receives identification information about a desired substrate for purchase from a user terminal, such as a smartphone, and identifies the location of the desired substrate for purchase on a store shelf based on the received identification information.

Patent Literature 2 indicated below discloses technology involving attaching an accessory equipped with a communication function to an inhaler device not having a communication function and performing communication between the inhaler device and another device via the accessory.

### Citation List

### Patent Literature

Patent Literature 1: Japanese Patent No. 6691138
Patent Literature 2: PCT International Publication No. WO 2016/156609

### Summary of Invention

### Technical Problem

It is conceivable that a user owns a plurality of accessories and uses an inhaler device while switching between the plurality of accessories in a replaceable manner. Such a usage case is not considered whatsoever in Patent Literatures mentioned above.

The present invention has been made in view of the problem mentioned above, and an object of the present invention is to provide a mechanism that can avoid an inconvenience that may occur when a plurality of accessories are replaced in an inhaler device.

### Solution to Problem

In order to solve the above problem, an aspect of the present invention provides a system including a plurality of accessories that are attachable to and detachable from an inhaler device and from and into which information is read and written via wireless communication, and a terminal device that stores synchronization information serving as information to be stored in common in the plurality of accessories and that writes the stored synchronization information into each of the plurality of accessories via the wireless communication. The inhaler device uses a substrate to generate an aerosol to be inhaled by a user

In a case where the synchronization information stored in the terminal device is newer than the synchronization information stored in the accessory, the terminal device may write the synchronization information stored in the terminal device into the accessory.

In a case where the synchronization information stored in the accessory is newer than the synchronization information stored in the terminal device, the terminal device may acquire and store information stored in the accessory as the synchronization information.

The terminal device may store the synchronization information and time information indicating a time point in association with each other and write the synchronization information and the time information in association with each other into the accessory. The terminal device may compare the time information stored in the terminal device and the time information stored in the accessory with each other so as to determine whether or not to write the synchronization information stored in the terminal device into the accessory.

The time information may indicate the time point of acquisition of the synchronization information.

The terminal device may store the accessory having written therein the synchronization information stored in the terminal device and write the synchronization information stored in the terminal device into the accessory not yet having written therein the synchronization information stored in the terminal device. The accessory not yet having written therein the synchronization information stored in the terminal device is any of the plurality of accessories.

The terminal device may write the synchronization information stored in the terminal device into the accessory attached to the inhaler device. The accessory attached to the inhaler device is any of the plurality of accessories.

The plurality of accessories may each be classified into any of a plurality of groups. The terminal device may write the synchronization information to be stored in common in a plurality of the accessories belonging to an identical group into each accessory belonging to the group.

Each accessory may include an NFC tag from and into which the information is read and written via the wireless communication.

The synchronization information may include identification information about the substrate.

The synchronization information may at least include either identification information about the inhaler device or identification information about the user using the inhaler device.

The synchronization information may include profile information indicating a profile that defines operation involving the inhaler device heating the substrate for generating the aerosol.

In order to solve the above problem, another aspect of the present invention provides a terminal device including a communicator that performs wireless communication with another device, a memory that stores synchronization information serving as information to be stored in common in a plurality of accessories that are attachable to and detachable from an inhaler device and from and into which information is read and written via the wireless communication, and a controller that controls a writing process involving writing the synchronization information stored in the memory into each of the plurality of accessories via the wireless communication by the communicator. The inhaler device uses a substrate to generate an aerosol to be inhaled by a user.

In order to solve the above problem, another aspect of the present invention provides a control method executed by a terminal device that performs wireless communication with another device. The control method includes controlling a writing process involving writing synchronization information into each of a plurality of accessories via the wireless communication. The synchronization information serves as information to be stored in common in the plurality of accessories that are attachable to and detachable from an inhaler device and from and into which information is read and written via the wireless communication. The inhaler device uses a substrate to generate an aerosol to be inhaled by a user.

In order to solve the above problem, another aspect of the present invention provides a program causing a computer to function as a controller. The computer controls a terminal device that performs wireless communication with another device. The controller controls a writing process involving writing synchronization information into each of a plurality of accessories via the wireless communication. The synchronization information serves as information to be stored in common in the plurality of accessories that are attachable to and detachable from an inhaler device and from and into which information is read and written via the wireless communication. The inhaler device uses a substrate to generate an aerosol to be inhaled by a user.

### Advantageous Effects of Invention

As described above, the present invention provides a mechanism that can avoid an inconvenience that may occur when a plurality of accessories are replaced in an inhaler device.

### Brief Description of Drawings

[Fig. 1] Fig. 1 is a schematic diagram of an inhaler device according to a first configuration example.
[Fig. 2] Fig. 2 is a schematic diagram of an inhaler device according to a second configuration example.
[Fig. 3] Fig. 3 illustrates an example of the configuration of a distribution system according to an embodiment.
[Fig. 4] Fig. 4 illustrates an example of the configuration of a user terminal according to this embodiment.
[Fig. 5] Fig. 5 illustrates an example of an accessory to be attached to an inhaler device 100 according to the second configuration example of this embodiment.
[Fig. 6] Fig. 6 illustrates an example of the configuration of a changing system according to this embodiment.
[Fig. 7] Fig. 7 illustrates a specific example of a synchronization process according to this embodiment.
[Fig. 8] Fig. 8 illustrates a specific example of the synchronization process according to this embodiment.
[Fig. 9] Fig. 9 illustrates a specific example of the synchronization process according to this embodiment.
[Fig. 10] Fig. 10 illustrates a specific example of the synchronization process according to this embodiment.
[Fig. 11] Fig. 11 is a flowchart illustrating an example of the flow of the synchronization process executed in the user terminal according to this embodiment.

### Description of Embodiments

Preferred embodiments of the present invention will be described in detail below with reference to the appended drawings. In this description and the drawings, structural elements having substantially identical functional configurations will be given the same reference signs, and redundant descriptions thereof will be omitted.

Furthermore, in this description and the drawings, elements having substantially identical functional configurations may sometimes be differentiated from each other by adding a different alphabet to the suffix of the same reference sign. For example, a plurality of elements having substantially identical functional configurations are differentiated from each other as in inhaler devices 100A and 100B, where necessary. However, if it is not necessary to particularly differentiate a plurality of elements having substantially identical functional configurations from each other, the same reference sign will be used. For example, if the inhaler devices 100A and 100B do not particularly need to be differentiated from each other, they will simply be referred to as "inhaler devices 100".

### 1. Configuration example of inhaler device

An inhaler device generates material to be inhaled by a user. In the example described below, the material generated by the inhaler device is an aerosol. Alternatively, the material generated by the inhaler device may be gas.

### (1) First configuration example

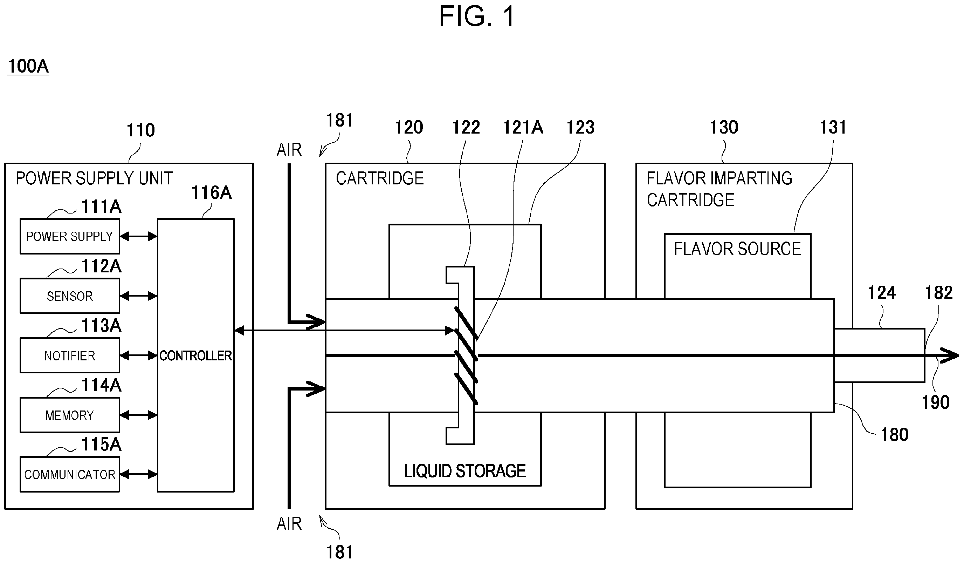

Fig. 1 is a schematic diagram of the inhaler device according to the first configuration example. As illustrated in Fig. 1, an inhaler device 100A according to the present configuration example includes a power supply unit 110, a cartridge 120, and a flavor imparting cartridge 130. The power supply unit 110 includes a power supply 111A, a sensor 112A, a notifier 113A, a memory 114A, a communicator 115A, and a controller 116A. The cartridge 120 includes a heater 121A, a liquid guide 122, and a liquid storage 123. The flavor imparting cartridge 130 includes a flavor source 131 and a mouthpiece 124. In the cartridge 120 and the flavor imparting cartridge 130, an airflow path 180 is defined.

The power supply 111A stores electric power. The power supply 111A supplies electric power to the structural elements of the inhaler device 100A under the control of the controller 116A. The power supply 111A may be a rechargeable battery such as a lithium ion secondary battery.

The sensor 112A acquires various items of information regarding the inhaler device 100A. In an example, the sensor 112A may be a pressure sensor such as a microphone condenser, a flow sensor, or a temperature sensor, and acquire a value generated in accordance with the user's inhalation. In another example, the sensor 112A may be an input device that receives information input by the user, such as a button or a switch.

The notifier 113A provides information to the user. The notifier 113A may be a light-emitting device that emits light, a display device that displays an image, a sound output device that outputs sound, or a vibration device that vibrates.

The memory 114A stores various items of information for operation of the inhaler device 100A. The memory 114A may be a non-volatile storage medium such as flash memory.

The communicator 115A is a communication interface capable of communication in conformity with any wired or wireless communication standard. Such a communication standard may be, for example, Wi-Fi (registered trademark) or Bluetooth (registered trademark).

The controller 116A functions as an arithmetic processing unit and a control circuit, and controls the overall operations of the inhaler device 100A in accordance with various programs. The controller 116A includes an electronic circuit such as a central processing unit (CPU) or a microprocessor, for example.

The liquid storage 123 stores an aerosol source. The aerosol source is atomized to generate an aerosol. The aerosol source is a liquid such as polyhydric alcohol and water. Examples of the polyhydric alcohol include glycerine and propylene glycol. The aerosol source may include a flavor component that is either derived from tobacco or not derived from tobacco. For the inhaler device 100A that is a medical inhaler such as a nebulizer, the aerosol source may include a medicine.

The liquid guide 122 guides, from the liquid storage 123, the aerosol source that is the liquid stored in the liquid storage 123, and holds the aerosol source. The liquid guide 122 is, for example, a wick formed by twining fiber material such as glass fiber or porous material such as porous ceramic. In this case, the capillary action of the wick guides the aerosol source stored in the liquid storage 123.

The heater 121A heats the aerosol source to atomize the aerosol source and generate the aerosol. In the example illustrated in Fig. 1, the heater 121A includes a coil wound around the liquid guide 122. When the heater 121A produces heat, the aerosol source held by the liquid guide 122 is heated and atomized to generate the aerosol. The heater 121A produces heat when receiving electric power from the power supply 111A. In an example, the electric power may be supplied in response to the sensor 112A detecting a start of the user's inhalation and/or an input of predetermined information. Subsequently, the supply of the electric power may be stopped in response to the sensor 112A detecting an end of the user's inhalation and/or an input of predetermined information.

The flavor source 131 is a structural element for imparting a flavor component to the aerosol. The flavor source 131 may include a flavor component that is either derived from tobacco or not derived from tobacco.

The airflow path 180 is a flow path of air to be inhaled by the user. The airflow path 180 has a tubular structure having an air inlet hole 181 and an air outlet hole 182 at both ends. The air inlet hole 181 is an inlet of air into the airflow path 180, and the air outlet hole 182 is an outlet of the air from the airflow path 180. The liquid guide 122 is on the airflow path 180 at an upstream position (closer to the air inlet hole 181), and the flavor source 131 is on the airflow path 180 at a downstream position (closer to the air outlet hole 182). Air flowing in through the air inlet hole 181 when the user inhales mixes with the aerosol generated by the heater 121A. Subsequently, as indicated by an arrow 190, the mixture fluid of the aerosol and the air passes through the flavor source 131 and is conveyed to the air outlet hole 182. When the mixture fluid of the aerosol and the air passes through the flavor source 131, the flavor component included in the flavor source 131 is imparted to the aerosol.

The mouthpiece 124 is to be held in a mouth of the user during inhalation. The mouthpiece 124 has the air outlet hole 182. When the user inhales with the mouthpiece 124 in his/her mouth, the mixture fluid of the aerosol and the air enters the oral cavity of the user

The configuration example of the inhaler device 100A has been described above. The inhaler device 100A is not limited to the above configuration, and may be configured in various ways as exemplified below.

In an example, the inhaler device 100A does not have to include the flavor imparting cartridge 130. In this case, the cartridge 120 includes the mouthpiece 124.

In another example, the inhaler device 100A may include various types of aerosol sources. Still another type of aerosol may be generated by mixing a plurality of types of aerosols generated from the plurality of types of aerosol sources in the airflow path 180 and causing a chemical reaction.

In addition, means for atomizing the aerosol source is not limited to heating by the heater 121A. For example, the means for atomizing the aerosol source may be vibration atomization or induction heating.

### (2) Second configuration example

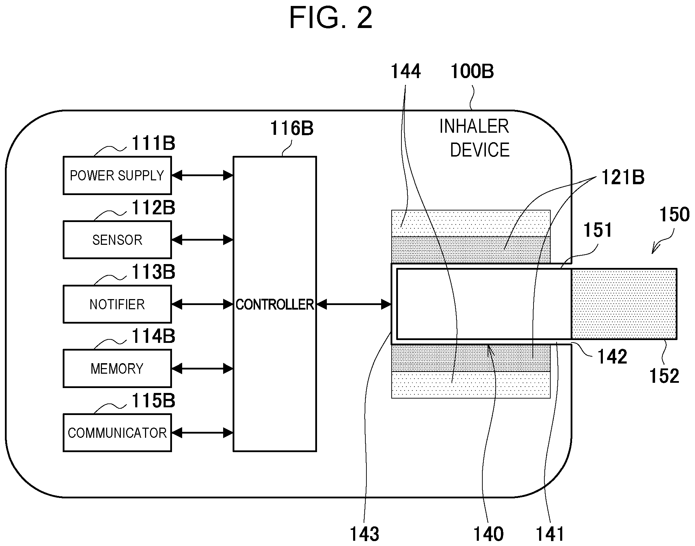

Fig. 2 is a schematic diagram of the inhaler device according to the second configuration example. As illustrated in Fig. 2, an inhaler device 100B according to the present configuration example includes a power supply 111B, a sensor 112B, a notifier 113B, a memory 114B, a communicator 115B, a controller 116B, a heater 121B, a holder 140, and a heat insulator 144.

The power supply 111B, the sensor 112B, the notifier 113B, the memory 114B, the communicator 115B, and the controller 116B are substantially the same as the respective corresponding structural elements included in the inhaler device 100A according to the first configuration example.

The holder 140 has an internal space 141, and holds a stick substrate 150 in a manner partially accommodated in the internal space 141. The holder 140 has an opening 142 that allows the internal space 141 to communicate with outside. The holder 140 accommodates the stick substrate 150 that is inserted into the internal space 141 through the opening 142. For example, the holder 140 may be a tubular body having the opening 142 and a bottom 143 on its ends, and may define the pillar-shaped internal space 141. The holder 140 also has a function for defining a flow path of air to be supplied to the stick substrate 150. For example, an air inlet hole serving as an inlet for the air toward the aforementioned flow path is disposed at the bottom 143. On the other hand, an air outlet hole serving as an outlet for the air from the aforementioned flow path is the opening 142.

The stick substrate 150 includes a substrate 151 and an inhalation port 152. The substrate 151 includes an aerosol source. In this configuration example, the aerosol source is not limited to a liquid and may alternatively be a solid. The stick substrate 150 held by the holder 140 includes the substrate 151 at least partially accommodated in the internal space 141 and the inhalation port 152 at least partially protruding from the opening 142. When the user inhales with the inhalation port 152 protruding from the opening 142 in his/her mouth, air flows into the internal space 141 through the air inlet hole (not illustrated), and the air and an aerosol generated from the substrate 151 reach inside the mouth of the user.

The heater 121B has a configuration similar to that of the heater 121A according to the first configuration example. In the example illustrated in Fig. 2, the heater 121B has a film-like shape and surrounds the outer circumference of the holder 140. Subsequently, heat produced from the heater 121B heats the substrate 151 of the stick substrate 150 from the outer circumference, generating the aerosol.

The heat insulator 144 prevents heat from transferring from the heater 121B to the other structural elements. For example, the heat insulator 144 may be a vacuum heat insulator or an aerogel heat insulator.

The configuration example of the inhaler device 100B has been described above. The inhaler device 100B is not limited to the above configuration, and may be configured in various ways as exemplified below.

In an example, the heater 121B may have a blade-like shape, and may be disposed so that the heater 121B protrudes from the bottom 143 of the holder 140 toward the internal space 141. In this case, the heater 121B having the blade-like shape is inserted into the substrate 151 of the stick substrate 150 and heats the substrate 151 of the stick substrate 150 from its inside. In another example, the heater 121B may be disposed so that the heater 121B covers the bottom 143 of the holder 140. In still another example, the heater 121B may be implemented as a combination of two or more selected from a first heater that covers the outer circumference of the holder 140, a second heater having the blade-like shape, and a third heater that covers the bottom 143 of the holder 140.

In another example, the holder 140 may include an opening/closing mechanism that at least partially opens and closes an outer shell defining the internal space 141. Examples of the opening/closing mechanism include a hinge. In addition, the holder 140 may sandwich the stick substrate 150 inserted into the internal space 141 by opening and closing the outer shell. In this case, the heater 121B may be at the sandwiching position of the holder 140 and may produce heat while pressing the stick substrate 150.

In addition, means for atomizing the aerosol source is not limited to heating by the heater 121B. For example, the means for atomizing the aerosol source may be induction heating.

In addition, the inhaler device 100B may also include the heater 121A, the liquid guide 122, the liquid storage 123, and the airflow path 180 according to the first configuration example, and the air outlet hole 182 of the airflow path 180 may also serve as an air inlet hole for the internal space 141. In this case, a mixture fluid of the air and an aerosol generated by the heater 121A flows into the internal space 141, mixes further with an aerosol generated by the heater 121B, and then reaches the oral cavity of the user.

### 2. Technical features

### 2.1. System configuration example

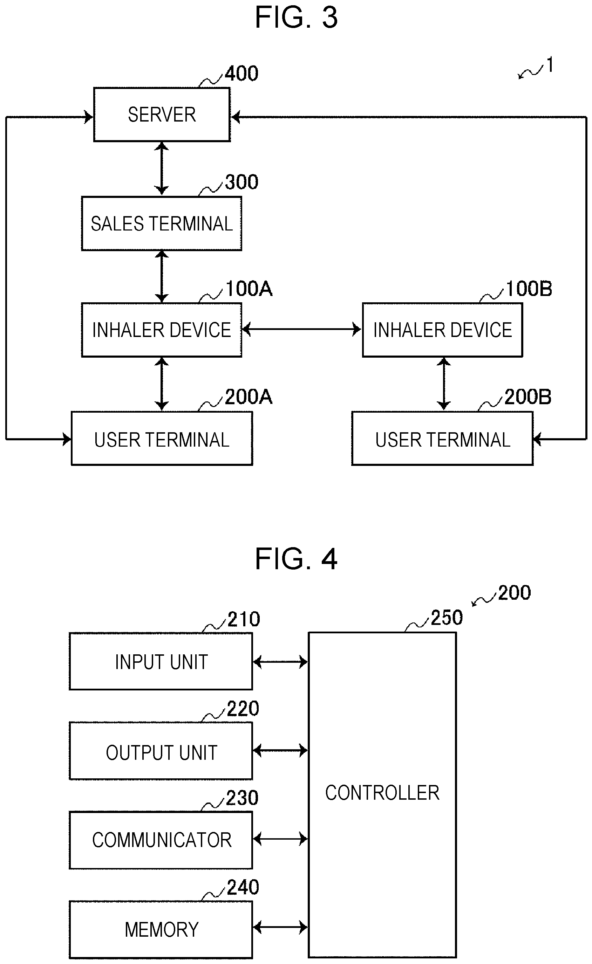

Fig. 3 illustrates an example of the configuration of a distribution system 1 according to this embodiment. As illustrated in Fig. 3, the distribution system 1 includes the inhaler devices 100, user terminals 200, a sales terminal 300, and a server 400. Although Fig. 3 illustrates an example where the distribution system 1 includes two inhaler devices 100 (100A and 100B), two user terminals 200 (200A and 200B), a single sales terminal 300, and a single server 400, the number of devices included in the distribution system 1 is not limited to this example.

### (1) Configuration of each inhaler device 100

An inhaler device generates material to be inhaled by a user. The material generated by the inhaler device is described below as being an aerosol. Alternatively, the material generated by the inhaler device may be a solid. The act of the user inhaling the material generated by the inhaler device may also be referred to as "inhalation" or "puff" hereinafter.

Each inhaler device 100 according to this embodiment uses a substrate to generate an aerosol to be inhaled by the user. A heater 121 is an example of a generator that generates an aerosol. The cartridge 120 and the flavor imparting cartridge 130 according to the first configuration example and the stick substrate 150 according to the second configuration example are examples of the substrate according to the present invention. The inhaler device 100 generates an aerosol by using the substrate attached to the inhaler device 100. In the first configuration example, the cartridge 120 and the flavor imparting cartridge 130 connected to the power supply unit 110 are examples of the substrate attached to the inhaler device 100. In the second configuration example, the stick substrate 150 fitted in the inhaler device 100 is an example of the substrate attached to the inhaler device 100.

The inhaler device 100 may have any configuration example selected from the first configuration example or the second configuration example described above. In Fig. 3, the inhaler device 100 to be used by a user A is the inhaler device 100A, and the inhaler device 100 to be used by a user B is the inhaler device 100B. In other words, the configuration of the inhaler device 100A in Fig. 3 does not necessarily have to match the configuration illustrated in Fig. 1, and may match the configuration illustrated in, for example, Fig. 2. Likewise, the configuration of the inhaler device 100B in Fig. 3 does not necessarily have to match the configuration illustrated in Fig. 2, and may match the configuration illustrated in, for example, Fig. 1.

A communicator 115 according to this embodiment is capable of performing wireless communication in conformity with a short-range wireless communication standard, such as NFC (near field communication) or Bluetooth (registered trademark).

### (2) Configuration of each user terminal 200

Each user terminal 200 is a terminal device used by the user of the corresponding inhaler device 100. For example, the user terminal 200 may be a smartphone, a tablet terminal, or a wearable device. The inhaler device 100 and the user terminal 200 that are used by the same user are associated with each other. For example, the user may register information about the inhaler device 100 in the user terminal 200, register information about the user terminal 200 in the inhaler device 100, or register information indicating that the inhaler device 100 and the user terminal 200 are used by the same user in the server 400. The configuration of the user terminal 200 will be described below with reference to Fig. 4.

Fig. 4 is a block diagram illustrating an example of the configuration of the user terminal 200 according to this embodiment. As illustrated in Fig. 4, the user terminal 200 includes an input unit 210, an output unit 220, a communicator 230, a memory 240, and a controller 250.

The input unit 210 has a function for receiving various types of information. The input unit 210 may include an input device that receives information input by the user. Examples include a button, a keyboard, a touchscreen, and a microphone. The input unit 210 may also include various types of sensors, such as an image sensor

The output unit 220 has a function for outputting information. The output unit 220 may include an output device that outputs information to the user. Examples of the output device include a display device that displays information, a light-emitting device that emits light, a vibrating device that vibrates, and a sound output device that outputs sound. An example of the display device is a display. An example of the light-emitting device is an LED (light-emitting diode). An example of the vibrating device is an eccentric motor. An example of the sound output device is a loudspeaker. The output unit 220 outputs information input from the controller 250 so as to notify the user of the information.

The communicator 230 is a communication interface for exchanging information between the user terminal 200 and another device. The communicator 230 performs communication in conformity with any wired or wireless communication standard. The communication standard used may be, for example, a wireless LAN (local area network), a wired LAN, Wi-Fi (registered trademark), or Bluetooth (registered trademark). In particular, the communicator 230 may perform wireless communication in conformity with a short-range wireless communication standard, such as NFC (near field communication) or Bluetooth (registered trademark).

The memory 240 stores various types of information for the operation of the user terminal 200. The memory 240 is a nonvolatile storage medium, such as a flash memory.

The controller 250 functions as an arithmetic processing unit and a control circuit, and controls the overall operation of the user terminal 200 in accordance with various programs. The controller 250 is implemented by, for example, an electronic circuit, such as a CPU (central processing unit) and a microprocessor. Furthermore, the controller 250 may include a ROM (read-only memory) that stores, for example, programs and arithmetic parameters to be used, and a RAM (random access memory) that temporarily stores parameters that change, as appropriate. The user terminal 200 executes various types of processing based on control by the controller 250. Processing of information input by the input unit 210, output of information by the output unit 220, exchanging of information by the communicator 230, and storing and reading of information by the memory 240 are examples of processing controlled by the controller 250. Other processing executed by the user terminal 200, such as processing based on information input to each structural element and information output from each structural element, is also controlled by the controller 250.

The function of the controller 250 may be implemented by using an application. The application may be preinstalled or may be downloaded. Alternatively, the function of the controller 250 may be implemented by using PWA (progressive web application).

### (3) Configuration of sales terminal 300

The sales terminal 300 is a terminal device that performs substrate sales processing. For example, the sales terminal 300 may be a cash register or a POS (point-of-sales) terminal. In that case, the sales terminal 300 is installed in a store that sells substrates. Examples of the substrate sales processing include accounting processing related to exchanging of money and sales management processing involving registering the brand and the number of sold substrates. A user using the sales terminal 300 is normally a store clerk.

The sales terminal 300 performs communication with another device in conformity with any wired or wireless communication standard. In one example, the sales terminal 300 may be capable of performing wireless communication in conformity with a short-range wireless communication standard, such as NFC (near field communication) or Bluetooth (registered trademark).

### (4) Configuration of server 400

The server 400 is an information processor that provides various types of services related to the inhaler devices 100. For example, the server 400 provides a membership service to the user of each inhaler device 100. The server 400 collects and accumulates information (i.e., information stored in a front panel 160 to be described later) about each inhaler device 100, analyzes the accumulated information, and provides the membership service in accordance with the analysis result. For example, each user accesses the server 400 to register his/her membership, so as to be able to receive various types of membership services, such as receive provided information about the corresponding inhaler device 100.

### 2.2. Features related to attachment of accessory

### (1) Configuration of front panel

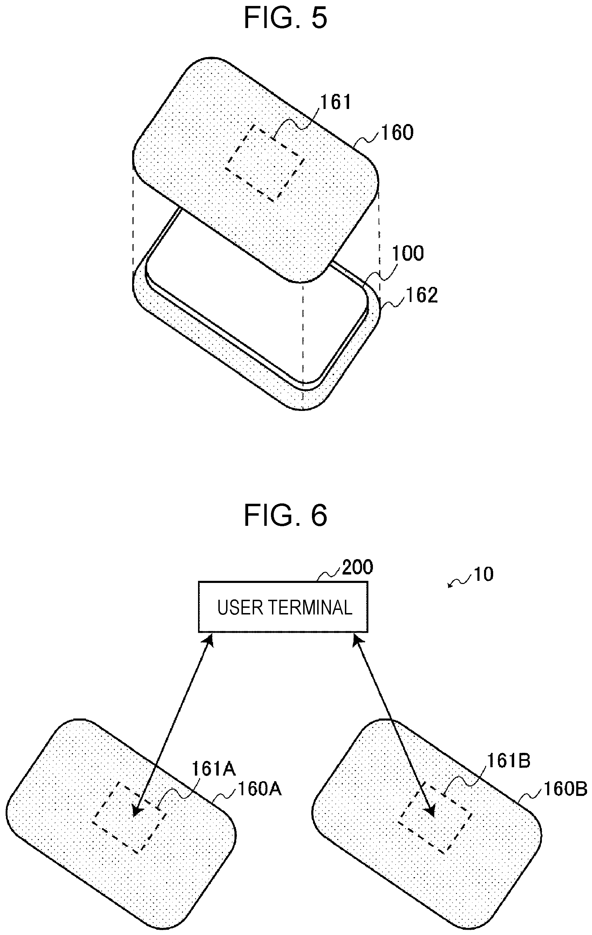

An accessory to be detachably attached to each inhaler device 100 may be attached to the inhaler device 100. Fig. 5 illustrates an example of the accessory to be attached to the inhaler device 100 according to the second configuration example of this embodiment. As illustrated in Fig. 5, a front panel 160 may be detachably attached to the inhaler device 100. The front panel 160 and a back panel 162 are joined to each other with the inhaler device 100 interposed therebetween, so as to form an outer shell that surrounds the outer periphery of the inhaler device 100. The front panel 160 is an example of the accessory.

The front panel 160 includes an NFC tag 161. The NFC tag 161 has both a wireless communication function and a storage function, and reads and writes information via wireless communication. Specifically, the NFC tag 161 performs NFC-based wireless communication with an NFC reader-writer to transmit information to the NFC reader-writer and to store information received from the NFC reader-writer. Because NFC does not require pairing, high usability is exhibited when wireless communication is performed with an unspecified device.

### (2) Processing of each device related to front panel

### (2.1) Processing by user terminal 200

The user terminal 200 manages information stored in the front panel 160. In one example, the user terminal 200 synchronizes with the NFC tag 161. Specifically, the user terminal 200 writes information into the NFC tag 161 or reads information from the NFC tag 161 and stores the information, such that the information stored in the NFC tag 161 and the information stored in the user terminal 200 match each other. The information synchronized in this manner may also be referred to as "synchronization information" hereinafter. The function for managing the information stored in the NFC tag 161 may be implemented by using a dedicated application (also referred to as "management application" hereinafter).

An example of the synchronization information will be described below. The synchronization information includes at least the information described below.

### Brand information

The synchronization information may include identification information about the substrate. The identification information about the substrate is used for identifying the brand (i.e., the type) of the substrate. The identification information about the substrate may also be referred to as "brand information" hereinafter. In a case where the brand information is transmitted to the sales terminal 300, the brand information is treated as information indicating the desired brand for purchase. Therefore, the transmission of the brand information as the synchronization information to the sales terminal 300 enables enhanced usability at the time of purchase of the substrate.

For example, the user terminal 200 may acquire the brand information from the membership service provided by the server 400 or from a website and write the brand information into the front panel 160. In another example, the user terminal 200 may write the brand information acquired by applying image recognition to a captured image of the package of the substrate into the front panel 160. The user terminal 200 may similarly acquire other information to be described below from the membership service or a web service and write the information into the front panel 160.

### Profile information

The synchronization information may include profile information indicating a profile that defines substrate heating operation performed by the inhaler device 100 for generating an aerosol. The inhaler device 100 generates an aerosol by heating the substrate in accordance with the profile indicated in the profile information.

The profile in the first configuration example is information that defines the time period in which electricity is supplied to the heater 121A and the amount of electricity (e.g., voltage) to be supplied thereto per unit time period. The controller 116 performs control such that the amount of electricity to be supplied per unit time period defined in the profile is supplied from the power supply 111 to the heater 121A for the electricity-supplying time period defined in the profile. For example, such electricity-supplying control is performed every time a puff is detected.

The profile in the second configuration example is information that defines the relationship between the time period elapsed from the start of heating by the heater 121B and the temperature of the heater 121B. The controller 116 controls the heater 121B such that a temperature change similar to a temperature change in the profile is realized in the heater 121B. The heater 121B may include a conductive track including a resistor, and the sensor 112 may detect the temperature of the heater 121B based on the electrical resistance of the conductive track. The control of the heater 121B may be implemented by controlling, for example, the electricity supplied from the power supply 111 to the heater 121B. The electricity-supplying control may be performed in accordance with, for example, PWM (pulse width modulation) control.

The profile information may be the profile itself. With regard to the profile, for example, a profile settable by being provided by the membership service may be set in advance. In that case, the profile information may be identification information indicating the profile. The identification information indicating the profile contains a smaller amount of data, as compared with the profile itself, so that the communication load can be reduced when the identification information is exchanged with another device. The profile may be customizable by the user. For example, a parameter, such as the electricity-supplying time period, may be adjusted by the user

### Combination of brand information and profile information

The brand information and the profile information may be associated with each other. For example, the profile preferred by the user may vary for every substrate, and the brand information and the profile suitable for the substrate of the brand indicated in the brand information may be associated with each other. Thus, the brand information and the profile information may be exchanged in association with each other as the synchronization information. Furthermore, the synchronization information may include identification information indicating a combination of the brand information and the profile information. The amount of data of the identification information indicating the combination of the brand information and the profile information is designed to be smaller than the total sum of the amount of data of the brand information and the amount of data of the profile information, so that the communication load can be reduced when the identification information is exchanged with another device. Examples of the identification information indicating the combination of the brand information and the profile information are indicated in Table 1 below.

### [Table 1]

**Table 1. Examples of identification information indicating combination of brand information and profile information**

| Identification Information | Brand Information | Profile Information | ... |
|---|---|---|---|
| 0001 | brand_X | P | ... |
| 0002 | brand_X | Q | ... |
| 0003 | brand_Y | P | |
| ... | ... | ... | ... |

### Device ID

The synchronization information may include identification information about the inhaler device 100. The identification information about the inhaler device 100 is used for uniquely identifying the inhaler device 100. The identification information about the inhaler device 100 may also be referred to "device ID" hereinafter.

### User ID

The synchronization information may include identification information about the user using the inhaler device 100. The identification information about the user is used for uniquely identifying the user. The identification information about the user may also be referred to as "user ID" hereinafter. The user ID may be personal information, such as the name of the user, or may be an account name registered in the membership service.

### Authentication information

The synchronization information may include authentication information. The authentication information indicates whether the user is qualified to purchase substrates. For example, the authentication information includes information for proving the age of the user

### (2.2) Processing by sales terminal 300

The sales terminal 300 reads information stored in the front panel 160 and performs various types of processing in accordance with the read information.

In one example, the sales terminal 300 may perform substrate selling processing in accordance with the information received from the inhaler device 100. For example, the sales terminal 300 may perform processing for selling the substrate of the brand indicated in the brand information received from the inhaler device 100 as the desired substrate for purchase by the user. With this configuration, the user can purchase the desired substrate for purchase without having to verbally designate the desired substrate to the store clerk.

In another example, the sales terminal 300 transmits the information received from the inhaler device 100 at least partially to the server 400. As will be described later, the server 400 accumulates and analyzes the received information and provides the membership service in accordance with the analysis result. Accordingly, the user can receive a preferred membership service that reflects the member's purchase behavior by simply purchasing a substrate by using the inhaler device 100, that is, without having to perform a troublesome process involving operating the user terminal 200 and transmitting information to the server 400.

In another example, the sales terminal 300 may perform authentication based on the authentication information received from the inhaler device 100. For example, the sales terminal 300 determines that the authentication is successful when the user's age indicated in the authentication information is above or equal to a predetermined age. Otherwise, the sales terminal 300 determines that the authentication has failed. This configuration can prevent a situation where a substrate is mistakenly sold to a user not qualified to purchase the substrate.

### (2.3) Processing by server 400

The server 400 collects information stored in the front panel 160 via the sales terminal 300. The server 400 accumulates and analyzes the collected information, and provides the membership service based on the analysis result.

In one example, the server 400 may compile the collected information for each item of brand information, so as to identify a brand that has been purchased in large numbers, that is, a brand that is popular among the members. Then, the server 400 may provide information for recommending the brand that is popular among the members as the membership service.

In another example, the server 400 may compile the collected information for each item of profile information, so as to identify a profile that has been used in large numbers, that is, a profile that is popular among the members. Then, the server 400 may provide information for recommending the profile that is popular among the members as the membership service.

In another example, an analysis for determining which brand's substrate has been sold to which user by the sales terminal 300, that is, which user has purchased which substrate, may be performed based on the combination of the user ID or the device ID and the brand information.

### (3) Changing of front panel 160

The user owns a plurality of front panels 160 and uses the inhaler device 100 with any one of the front panels 160 attached to the inhaler device 100. An example of this system will now be described with reference to Fig. 6.

Fig. 6 illustrates an example of the configuration of a changing system 10 according to this embodiment. As illustrated in Fig. 6, the changing system 10 includes a user terminal 200 and a plurality of front panels 160. The changing system 10 may also include an inhaler device 100 to which the plurality of front panels 160 are attachable. Each device included in the changing system 10 is normally owned by the same user. Either one of a front panel 160A and a front panel 160B may be attached to the inhaler device 100. The user can change the front panel 160 on the inhaler device 100 depending on the user's mood of the day.

If there is a difference between information stored in the front panel 160A (i.e., NFC tag 161A) and information stored in the front panel 160B (i.e., NFC tag 161B), various inconveniences may occur before and after the changing of the front panel. For example, if the brand information is different between the front panel 160A and the front panel 160B and the user purchases a substrate without recognizing the difference, the substrate of the brand unintentionally purchased by the user after the changing of the front panel is different from that before the changing of the front panel.

The user terminal 200 stores synchronization information serving as information to be stored in common in the plurality of front panels 160 so as to synchronize with the plurality of front panels 160. Specifically, the user terminal 200 writes the synchronization information stored in the user terminal 200 into each of the plurality of front panels 160 via wireless communication. In detail, the memory 240 stores the aforementioned synchronization information as information to be stored in common in the plurality of front panels 160. Then, the controller 250 controls a writing process involving writing the synchronization information stored in the memory 240 into each front panel 160 via wireless communication by the communicator 230. The writing process includes establishing NFC communication with each NFC tag 161 and transmitting the synchronization information. For example, the user terminal 200 writes identical synchronization information into both the NFC tag 161A and the NFC tag 161B. This configuration enables synchronization of information to be stored in the NFC tags 161 of the plurality of front panels 160, thereby avoiding the aforementioned inconveniences.

If the synchronization information stored in the user terminal 200 is newer than the synchronization information stored in each front panel 160, the user terminal 200 writes the synchronization information stored in the user terminal 200 into the front panel 160. With this configuration, information to be stored in each of the plurality of front panels 160 can be updated to the latest information.

If the synchronization information stored in each front panel 160 is newer than the synchronization information stored in the user terminal 200, the user terminal 200 acquires and stores the information stored in the front panel 160 as the synchronization information. There is a possibility that information is written into the front panel 160 from a device other than the user terminal 200 (e.g., another user terminal 200 used by another user). In this regard, this configuration allows the plurality of front panels 160 to synchronize information written by another device as the latest synchronization information.

There are various conceivable methods for determining which one of the synchronization information stored in the user terminal 200 and the synchronization information stored in each front panel 160 is the newer information. The following description relates to an example of the determination method based on time information and an example of the determination method based on a write history.

### Determination method based on time information

The user terminal 200 stores synchronization information and time information indicating a time point in association with each other and writes the synchronization information and the time information in association with each other into each front panel 160. Then, the user terminal 200 compares the time information stored in the user terminal 200 and the time information stored in the front panel 160 with each other so as to determine whether or not to write the synchronization information stored in the user terminal 200 into the front panel 160. In a case where the time point indicated in the time information stored in the user terminal 200 and the time point indicated in the time information stored in the front panel 160 match each other, such a case implies that the latest information is written in the front panel 160. In contrast, in a case where these time points differ from each other, such a case implies that one of the synchronization information stored in the user terminal 200 and the synchronization information stored in the front panel 160 is newer than the other.

Specifically, in a case where the time point indicated in the time information stored in the user terminal 200 is newer than the time point indicated in the time information stored in the front panel 160, such a case implies that old information is written in the front panel 160. In this case, the user terminal 200 writes the synchronization information stored in the user terminal 200 into the front panel 160.

In a case where the time point indicated in the time information stored in the front panel 160 is newer than the time point indicated in the time information stored in the user terminal 200, such a case implies that new information is written in the front panel 160. In this case, the user terminal 200 acquires and stores the synchronization information stored in the front panel 160.

With the above-described configuration, it is possible to determine which one of the synchronization information stored in the user terminal 200 and the synchronization information stored in the front panel 160 is newer. Thus, the user terminal 200 can update the synchronization information stored in either the user terminal 200 or the front panel 160 to the latest information. In other words, the user terminal 200 can synchronize with the front panel 160.

Time information indicates a time point of acquisition of synchronization information. In one example, time information associated with brand information indicates a time point at which the brand information is acquired by the user terminal 200 from a website or a package. The concept of the time point in this case includes the date and time. With this configuration, information newly acquired by the user terminal 200 can be stored in common in the plurality of front panels 160.

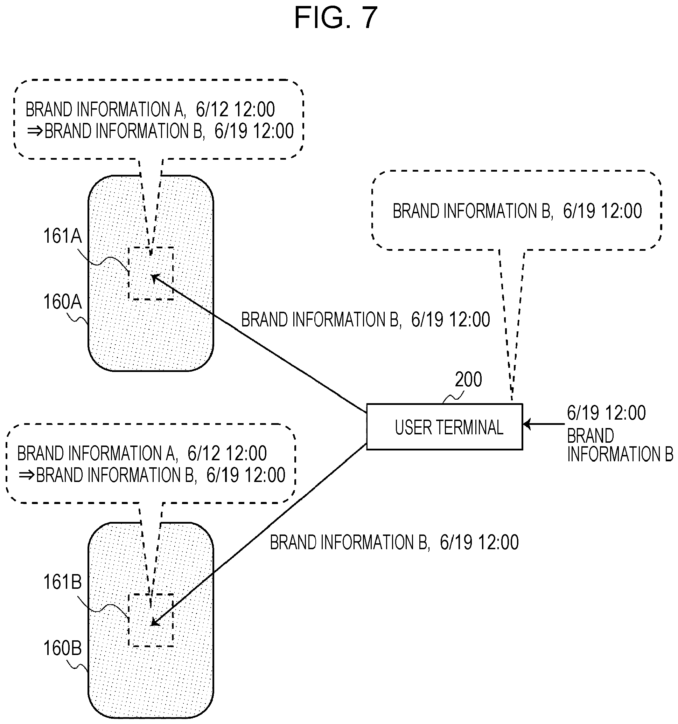

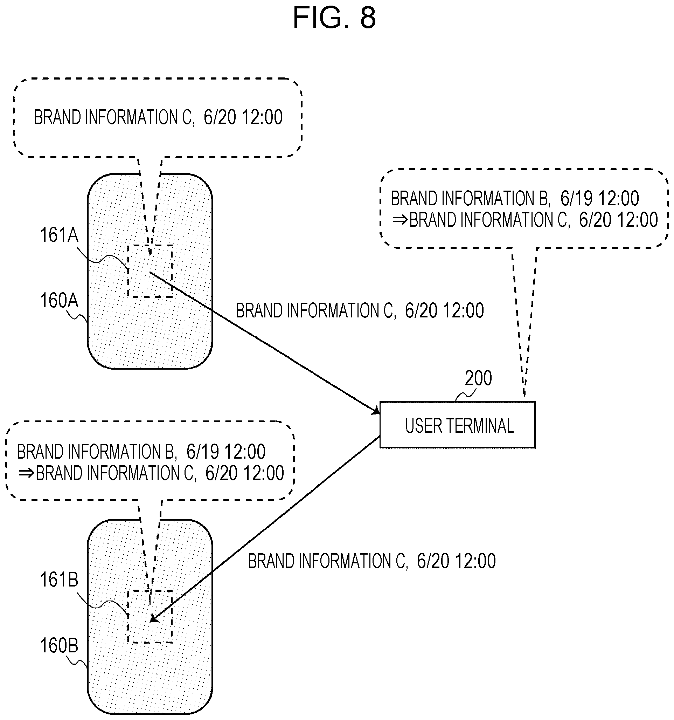

Specific examples of this determination method will be described below with reference to Fig. 7 and Fig. 8.

Fig. 7 illustrates a specific example of the synchronization process according to this embodiment. As illustrated in Fig. 7, the front panel 160A (i.e., the NFC tag 161A) and the front panel 160B (i.e., the NFC tag 161B) store brand information A in association with time information "6/12 12:00". When the user terminal 200 acquires brand information B from a website at 12:00 on June 19, the user terminal 200 stores the brand information B in association with time information "6/19 12:00". When the user terminal 200 reads the brand information A and the time information "6/12 12:00" from the front panel 160A, the user terminal 200 compares the read time information "6/12 12:00" and the stored time information "6/19 12:00" with each other and determines that the brand information B is the newer information. Then, the user terminal 200 writes the brand information B in association with the time information "6/19 12:00" into the front panel 160A. The user terminal 200 performs a similar process on the front panel 160B.

Fig. 8 illustrates a specific example of the synchronization process according to this embodiment. This specific example relates to a case where brand information C is written into the front panel 160A by another user terminal 200 after the specific example illustrated in Fig. 7. As illustrated in Fig. 8, the front panel 160A (i.e., the NFC tag 161A) stores the brand information C in association with time information "6/20 12:00". On the other hand, the front panel 160B (i.e., the NFC tag 161B) stores the brand information B in association with the time information "6/19 12:00". The user terminal 200 stores the brand information B in association with the time information "6/19 12:00". When the user terminal 200 reads the brand information C and the time information "6/20 12:00" from the front panel 160A, the user terminal 200 compares the read time information "6/20 12:00" and the stored time information "6/19 12:00" with each other and determines that the brand information C is the newer information. Then, the user terminal 200 stores the brand information C in association with the time information "6/20 12:00". On the other hand, when the user terminal 200 reads the brand information B and the time information "6/19 12:00" from the front panel 160B, the user terminals 200 compares the read time information "6/19 12:00" and the stored time information "6/20 12:00" with each other and determines that the brand information C is the newer information. Then, the user terminal 200 writes the brand information C in association with the time information "6/20 12:00" into the front panel 160B.

### Determination method based on write history

The user terminal 200 stores a front panel 160 having the synchronization information stored in the user terminal 200 written therein. For example, the user terminal 200 stores whether or not each of the plurality of front panels 160 has the synchronization information stored in the user terminal 200 written therein. Then, the user terminal 200 writes the synchronization information stored in the user terminal 200 into a front panel 160, of the plurality of front panels 160, not yet having written therein the synchronization information stored in the user terminal 200. With this configuration, information newly acquired by the user terminal 200 can be stored in common in the plurality of front panels 160.

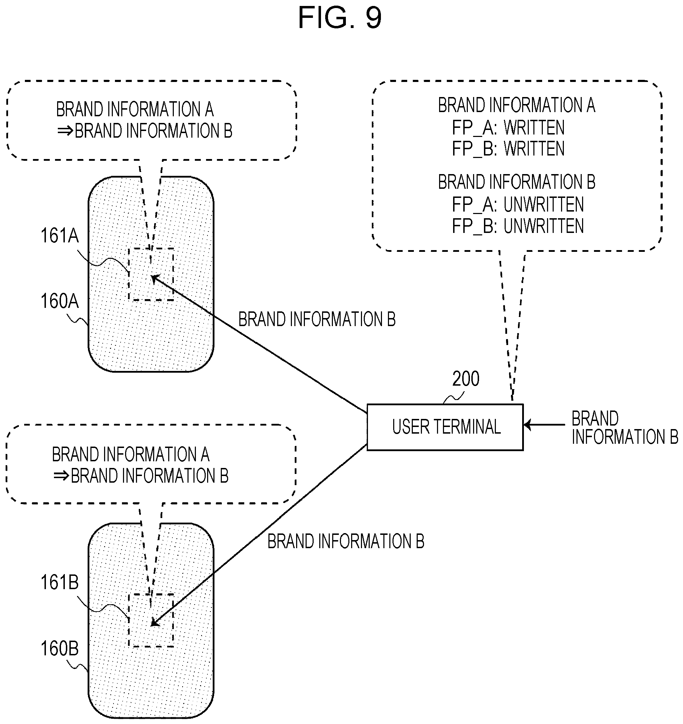

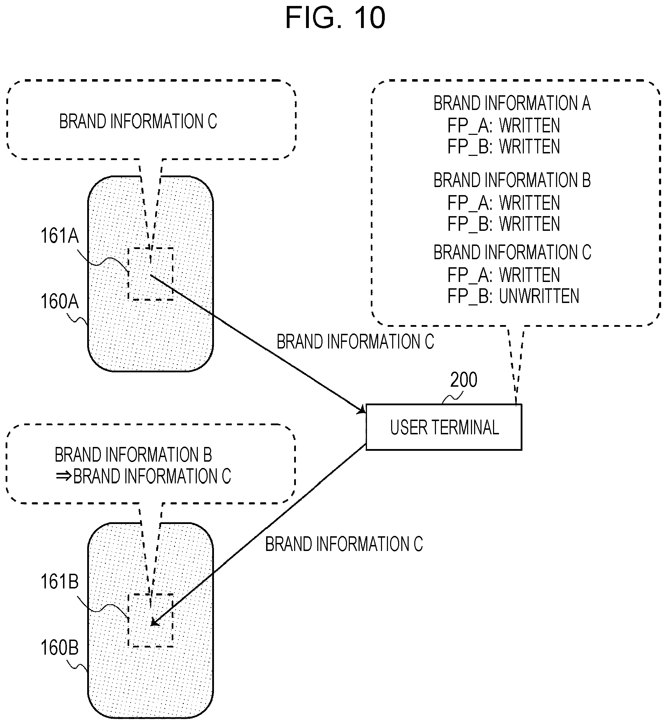

Specific examples of this determination method will be described below with reference to Fig. 9 and Fig. 10.

Fig. 9 illustrates a specific example of the synchronization process according to this embodiment. As illustrated in Fig. 9, the front panel 160A (i.e., the NFC tag 161A) and the front panel 160B (i.e., the NFC tag 161B) each store brand information A. The user terminal 200 stores, as a write history, information indicating that the brand information A is written in each of the front panel 160A and the front panel 160B. In Fig. 9, FP_A denotes the front panel 160A, and FP_B denotes the front panel 160B. The write history is stored when the brand information A is written into each of the front panel 160A and the front panel 160B in the past by the user terminal 200. When the user terminal 200 acquires brand information B from a website, the user terminal 200 stores the brand information B as synchronization information and stores, as a write history, information indicating that the brand information B is not written in each of the front panel 160A and the front panel 160B. Thus, the user terminal 200 writes the brand information B into the front panel 160A and stores, as a write history, information indicating that the brand information B is written in the front panel 160A. The user terminal 200 performs a similar process on the front panel 160B.

Fig. 10 illustrates a specific example of the synchronization process according to this embodiment. This specific example relates to a case where brand information C is written into the front panel 160A by another user terminal 200 after the specific example illustrated in Fig. 9. As illustrated in Fig. 10, the front panel 160A (i.e., the NFC tag 161A) has the brand information C stored therein. The front panel 160B (i.e., the NFC tag 161B) has the brand information B stored therein. The user terminal 200 stores, as a write history, information indicating that the brand information A and the brand information B are written in each of the front panel 160A and the front panel 160B. When the user terminal 200 reads the brand information C from the front panel 160A, the user terminal 200 determines that the brand information C is information newly written from another user terminal 200 based on the fact that the brand information C is not included in the write history. Then, the user terminal 200 stores the brand information C as synchronization information and stores, as a write history, information indicating that the brand information C is written in the front panel 160A and that the brand information C is not written in the front panel 160B. The user terminal 200 then writes the brand information C into the front panel 160B and stores, as a write history, information indicating that the brand information C is written in the front panel 160B.

### Synchronization timing

Of the plurality of front panels 160, the user terminal 200 synchronizes with a front panel 160 with whom the user terminal 200 has established NFC communication. In other words, the user terminal 200 synchronizes with the front panel 160 at a timing at which the NFC communication with the front panel 160 is established. For example, the user can allow the user terminal 200 and the front panel 160 to synchronize with each other by holding the user terminal 200 over the front panel 160.

Furthermore, the user terminal 200 may write the synchronization information stored in the user terminal 200 into the front panel 160, of the plurality of front panels 160, attached to the inhaler device 100. In other words, the user terminal 200 may synchronize with the front panel 160 at a timing at which the front panel 160 is attached to the inhaler device 100. With this configuration, synchronization is executed on the front panel 160 attached to the inhaler device 100, so that unwanted synchronization, such as synchronization with a front panel 160 that has not been used for a long period of time, can be avoided.

There are various conceivable methods for the user terminal 200 to identify the front panel 160 attached to the inhaler device 100 and to synchronize limitedly with the identified front panel 160. In one example, in a case where the inhaler device 100 and the front panel 160 are electrically connectable to each other, the sensor 112 may identify the front panel 160 attached to the inhaler device 100 based on the electrical resistance. In that case, the inhaler device 100 uses the communicator 115 to transmit the identification result of the front panel 160 to the user terminal 200. Accordingly, the user terminal 200 can synchronize limitedly with the front panel 160 identified in accordance with the received information.

### (4) Flow of process

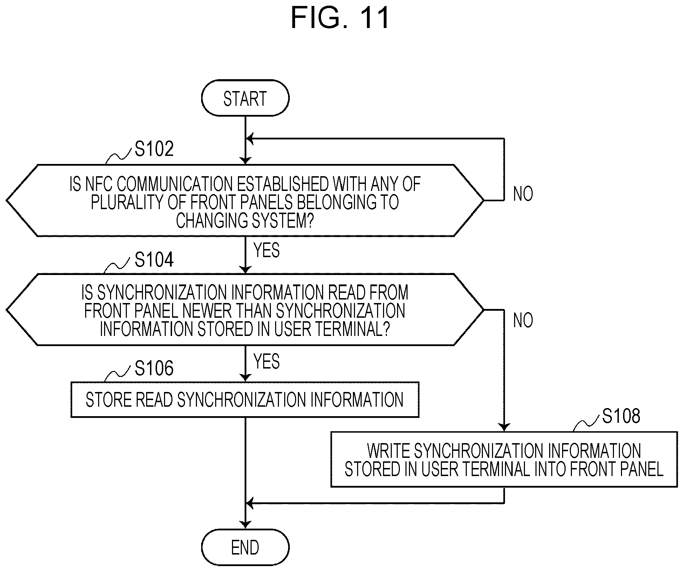

Fig. 11 is a flowchart illustrating an example of the flow of the synchronization process executed in the user terminal 200 according to this embodiment.

As illustrated in Fig. 11, the user terminal 200 first determines whether or not NFC communication is established with any of the plurality of front panels 160 belonging to the changing system 10 (step S102).

If it is determined that NFC communication is not established (step S102: NO), the user terminal 200 waits until the NFC communication is established.

On the other hand, if it is determined that NFC communication is established (step S102: YES), the user terminal 200 determines whether or not synchronization information read from the front panel 160 is newer than synchronization information stored in the user terminal 200 (step S104).

If it is determined that the synchronization information read from the front panel 160 is the newer information (step S104: YES), the user terminal 200 stores the read synchronization information (step S106).

On the other hand, if it is determined that the synchronization information stored in the user terminal 200 is the newer information (step S104: NO), the user terminal 200 writes the synchronization information stored in the user terminal 200 into the front panel 160 (step S108).

### 3. Supplemental remarks

Although preferred embodiments of the present invention have been described in detail above with reference to the appended drawings, the present invention is not limited to these examples. It is apparent to a person with a common knowledge of the technical field to which the present invention belongs that various modifications and alterations are conceivable within the scope of the technical ideas defined in the claims, and it is to be understood that such modifications and alterations naturally belong to the technical scope of the present invention.

The above-described embodiment relates an example where the user terminal 200 stores a single type of synchronization information and writes the synchronization information in common into the plurality of front panels 160. However, the present invention is not limited to such an example. The user terminal 200 may store a plurality of types of synchronization information. Then, for example, the user terminal 200 may write a single type of synchronization information selected by the user in common into the plurality of front panels 160. The plurality of front panels 160 belonging to the changing system 10 may each be classified into any of a plurality of groups. In that case, the user terminal 200 stores synchronization information for each group. Then, the user terminal 200 writes the synchronization information to be stored in common in a plurality of front panels 160 belonging to the same group into the front panels 160 belonging to the group. For example, the user terminal 200 stores private synchronization information and work synchronization information. The user terminal 200 writes the private synchronization information into each of a plurality of private front panels 160, and writes the work synchronization information into each of a plurality of work front panels 160. This configuration enables enhanced usability when the plurality of front panels 160 are properly used for the respective purposes.

The above-described embodiment relates to an example where each front panel 160 is equipped with an NFC tag 161. However, the present invention is not limited to such an example. The present invention is applicable to any accessory having a communication function and a storage function. The accessory may be equipped with a communication function in conformity with another wireless communication standard other than NFC. An example of another wireless communication standard is Bluetooth.

For example, although each front panel 160 is described as an example of an accessory to be attached to the inhaler device 100 in the above embodiment, the present invention is not limited to this example. The accessory may be any component, such as a strap or a sticker, attachable to and detachable from the inhaler device 100.

For example, although an example where an accessory is to be attached to the inhaler device 100 is described in the above embodiment, the present invention is not limited to this example. The present invention is applicable to any device other than the inhaler device 100. For example, an accessory, such as a smartphone case, may be detachably attached to a smartphone. The control of the smartphone may be switched when the accessory is attached or detached.

The series of processing by each device described in this description may be implemented by using any of software, hardware, and a combination of software and hardware. A program serving as software is stored in advance in a storage medium (non-transitory medium) provided inside or outside each device. For example, each program is loaded into a RAM at the time of execution by a computer, and is executed by a processor, such as a CPU. The aforementioned storage medium is, for example, a magnetic disk, an optical disk, a magneto-optical disk, or a flash memory. The aforementioned computer program may be distributed via, for example, a network without using a storage medium.

The processing described using the flowchart and the sequence diagram in this description does not necessarily have to be executed in the illustrated order. Some of the steps may be executed concurrently. Moreover, an additional step may be employed, and one or more of the steps may be omitted.

The following configurations also belong to the technical scope of the present invention.

### Item (1)

A system includes:
a plurality of accessories that are attachable to and detachable from an inhaler device and from and into which information is read and written via wireless communication, the inhaler device using a substrate to generate an aerosol to be inhaled by a user; and
a terminal device that stores synchronization information serving as information to be stored in common in the plurality of accessories and that writes the stored synchronization information into each of the plurality of accessories via the wireless communication.

### Item (2)

In the system according to item (1),
in a case where the synchronization information stored in the terminal device is newer than the synchronization information stored in the accessory, the terminal device writes the synchronization information stored in the terminal device into the accessory.

### Item (3)

In the system according to item (1) or (2),
in a case where the synchronization information stored in the accessory is newer than the synchronization information stored in the terminal device, the terminal device acquires and stores information stored in the accessory as the synchronization information.

### Item (4)

In the system according to item (2) or (3),
the terminal device stores the synchronization information and time information indicating a time point in association with each other and writes the synchronization information and the time information in association with each other into the accessory, and
the terminal device compares the time information stored in the terminal device and the time information stored in the accessory with each other so as to determine whether or not to write the synchronization information stored in the terminal device into the accessory.

### Item (5)

In the system according to item (4),
the time information indicates the time point of acquisition of the synchronization information.

### Item (6)

In the system according to item (1),
the terminal device stores the accessory having written therein the synchronization information stored in the terminal device and writes the synchronization information stored in the terminal device into the accessory not yet having written therein the synchronization information stored in the terminal device, the accessory not yet having written therein the synchronization information stored in the terminal device being any of the plurality of accessories.

### Item (7)

In the system according to any one of items (1) to (6),
the terminal device writes the synchronization information stored in the terminal device into the accessory attached to the inhaler device, the accessory attached to the inhaler device being any of the plurality of accessories.

### Item (8)

In the system according to any one of items (1) to (7),
the plurality of accessories are each classified into any of a plurality of groups, and
the terminal device writes the synchronization information to be stored in common in a plurality of the accessories belonging to an identical group into each accessory belonging to the group.

### Item (9)

In the system according to any one of items (1) to (8),
each accessory includes an NFC tag from and into which the information is read and written via the wireless communication.

### Item (10)

In the system according to any one of items (1) to (9),
the synchronization information includes identification information about the substrate.

### Item (11)

In the system according to any one of items (1) to (10),
the synchronization information at least includes either identification information about the inhaler device or identification information about the user using the inhaler device.

### Item (12)

In the system according to any one of items (1) to (11),
the synchronization information includes profile information indicating a profile that defines operation involving the inhaler device heating the substrate for generating the aerosol.

### Item (13)

A terminal device includes:
a communicator that performs wireless communication with another device;
a memory that stores synchronization information serving as information to be stored in common in a plurality of accessories that are attachable to and detachable from an inhaler device and from and into which information is read and written via the wireless communication, the inhaler device using a substrate to generate an aerosol to be inhaled by a user; and
a controller that controls a writing process involving writing the synchronization information stored in the memory into each of the plurality of accessories via the wireless communication by the communicator.

### Item (14)

A control method executed by a terminal device that performs wireless communication with another device includes:
controlling a writing process involving writing synchronization information into each of a plurality of accessories via the wireless communication, the synchronization information serving as information to be stored in common in the plurality of accessories that are attachable to and detachable from an inhaler device and from and into which information is read and written via the wireless communication, the inhaler device using a substrate to generate an aerosol to be inhaled by a user

### Item (15)

A program causes a computer to function as a controller. The computer controls a terminal device that performs wireless communication with another device.

The controller controls a writing process involving writing synchronization information into each of a plurality of accessories via the wireless communication, the synchronization information serving as information to be stored in common in the plurality of accessories that are attachable to and detachable from an inhaler device and from and into which information is read and written via the wireless communication, the inhaler device using a substrate to generate an aerosol to be inhaled by a user.

### Reference Signs List

- 1: distribution system
- 100: inhaler device
- 110: power supply unit
- 111: power supply
- 112: sensor
- 113: notifier
- 114: memory
- 115: communicator, main communicator
- 116: controller
- 120: cartridge
- 121: heater
- 122: liquid guide
- 123: liquid storage
- 124: mouthpiece
- 130: flavor imparting cartridge
- 131: flavor source
- 140: holder
- 141: internal space
- 142: opening
- 143: bottom
- 144: heat insulator
- 150: stick substrate
- 151: substrate
- 152: inhalation port
- 160: front panel
- 161: NFC tag
- 162: back panel
- 180: airflow path
- 181: air inlet hole
- 182: air outlet hole
- 200: user terminal
- 300: sales terminal
- 400: server

## Claims

1. A system comprising:
a plurality of accessories that are attachable to and detachable from an inhaler device and from and into which information is read and written via wireless communication, the inhaler device using a substrate to generate an aerosol to be inhaled by a user; and
a terminal device that stores synchronization information serving as information to be stored in common in the plurality of accessories and that writes the stored synchronization information into each of the plurality of accessories via the wireless communication.

2. The system according to claim 1,
wherein, in a case where the synchronization information stored in the terminal device is newer than the synchronization information stored in the accessory, the terminal device writes the synchronization information stored in the terminal device into the accessory.

3. The system according to claim 1 or 2,
wherein, in a case where the synchronization information stored in the accessory is newer than the synchronization information stored in the terminal device, the terminal device acquires and stores information stored in the accessory as the synchronization information.

4. The system according to claim 2 or 3,
wherein the terminal device stores the synchronization information and time information indicating a time point in association with each other and writes the synchronization information and the time information in association with each other into the accessory, and
wherein the terminal device compares the time information stored in the terminal device and the time information stored in the accessory with each other so as to determine whether or not to write the synchronization information stored in the terminal device into the accessory.

5. The system according to claim 4,
wherein the time information indicates the time point of acquisition of the synchronization information.

6. The system according to claim 1,
wherein the terminal device stores the accessory having written therein the synchronization information stored in the terminal device and writes the synchronization information stored in the terminal device into the accessory not yet having written therein the synchronization information stored in the terminal device, the accessory not yet having written therein the synchronization information stored in the terminal device being any of the plurality of accessories.

7. The system according to any one of claims 1 to 6,
wherein the terminal device writes the synchronization information stored in the terminal device into the accessory attached to the inhaler device, the accessory attached to the inhaler device being any of the plurality of accessories.

8. The system according to any one of claims 1 to 7,
wherein the plurality of accessories are each classified into any of a plurality of groups, and
wherein the terminal device writes the synchronization information to be stored in common in a plurality of the accessories belonging to an identical group into each accessory belonging to the group.

9. The system according to any one of claims 1 to 8,
wherein each accessory includes an NFC tag from and into which the information is read and written via the wireless communication.

10. The system according to any one of claims 1 to 9,
wherein the synchronization information includes identification information about the substrate.

11. The system according to any one of claims 1 to 10,
wherein the synchronization information at least includes either identification information about the inhaler device or identification information about the user using the inhaler device.

12. The system according to any one of claims 1 to 11,
wherein the synchronization information includes profile information indicating a profile that defines operation involving the inhaler device heating the substrate for generating the aerosol.

13. A terminal device comprising:
a communicator that performs wireless communication with another device;
a memory that stores synchronization information serving as information to be stored in common in a plurality of accessories that are attachable to and detachable from an inhaler device and from and into which information is read and written via the wireless communication, the inhaler device using a substrate to generate an aerosol to be inhaled by a user; and
a controller that controls a writing process involving writing the synchronization information stored in the memory into each of the plurality of accessories via the wireless communication by the communicator.

14. A control method executed by a terminal device that performs wireless communication with another device, the control method comprising:
controlling a writing process involving writing synchronization information into each of a plurality of accessories via the wireless communication, the synchronization information serving as information to be stored in common in the plurality of accessories that are attachable to and detachable from an inhaler device and from and into which information is read and written via the wireless communication, the inhaler device using a substrate to generate an aerosol to be inhaled by a user.

15. A program causing a computer to function as a controller, the computer controlling a terminal device that performs wireless communication with another device,
wherein the controller controls a writing process involving writing synchronization information into each of a plurality of accessories via the wireless communication, the synchronization information serving as information to be stored in common in the plurality of accessories that are attachable to and detachable from an inhaler device and from and into which information is read and written via the wireless communication, the inhaler device using a substrate to generate an aerosol to be inhaled by a user.
